(19) Europäisches Patentamt
European Patent Office
Office européen des brevets

(11) EP 4 116 900 A1

(12) EUROPEAN PATENT APPLICATION
published in accordance with Art. 153(4) EPC

(43) Date of publication:
11.01.2023 Bulletin 2023/02

(51) International Patent Classification (IPC):
G06Q 10/06 (2012.01)   G01N 35/00 (2006.01)

(21) Application number: 21763999.6

(52) Cooperative Patent Classification (CPC):
G01N 35/00; G06Q 10/06

(22) Date of filing: 19.01.2021

(86) International application number:
PCT/JP2021/001671

(87) International publication number:
WO 2021/176867 (10.09.2021 Gazette 2021/36)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 05.03.2020 JP 2020037545

(71) Applicant: Hitachi High-Tech Corporation
Minato-ku
Tokyo 105-6409 (JP)

(72) Inventors:
• SEKI, Yoshihiro
Tokyo 105-6409 (JP)
• KOIZUMI, Nobuhiro
Tokyo 105-6409 (JP)
• NAKAJIMA, Aika
Tokyo 105-6409 (JP)

(74) Representative: MERH-IP Matias Erny Reichl
Hoffmann
Patentanwälte PartG mbB
Paul-Heyse-Strasse 29
80336 München (DE)

(54) MANAGEMENT DEVICE AND REQUEST METHOD

(57) In order to appropriately manage reagent replacement and calibration/quality management processing for a plurality of analysis devices in a clinical laboratory unit, provided is a management device 102 configured to manage a plurality of analysis devices in a clinical laboratory 101 provided with the plurality of analysis devices and a cabinet that stores a reagent used for measurement by the plurality of analysis devices or a calibration/quality management sample used for calibration/quality management processing of the reagents, the management device 102 including: a reagent replacement preparation timing detection unit 206 configured to detect a timing to start reagent replacement preparation, based on a remaining amount of each of in-measurement reagents and a first preparation time required for preparation for replacing the reagent; calibration/quality management processing preparation timing detection units 207, 208 configured to detect a timing to start calibration/quality management processing preparation, based on the remaining amount of the in-measurement reagent and a second preparation time required for preparation for the calibration/quality management processing; and a notification person-in-charge determination unit 209 configured to notify a laboratory technician of a request for the reagent replacement preparation or the calibration/quality management processing preparation upon receiving detection of a timing by the detection units.

[FIG. 2]

**Description**

Technical Field

The present invention relates to a management device for managing a plurality of automatic analysis devices for analyzing a biological sample such as blood or urine and a request method for requesting preparation of reagent replacement and calibration/quality management processing for the plurality of automatic analysis devices in a clinical laboratory provided with the plurality of automatic analysis devices.

Background Art

An automatic analysis device that analyzes a biological sample such as blood or urine has merits such as high reproducibility of analysis quality and quickness of analysis processing, and thus is indispensable for current diagnosis. There are a plurality of types of automatic analysis devices according to types of analysis. Examples include a colorimetric analysis device that performs biochemical analysis, an immune analysis device that analyzes an antigen or antibody in a sample using an antigen-antibody reaction, a coagulation analysis device that measures coagulability of blood, a blood cell counter that measures the number of blood cell components in blood, and the like. When analyzing a sample by the automatic analysis device, a pre-processing device that performs pre-processing such as centrifugation of blood or dispensing of the sample to prepare a plurality of child samples is also used. In a single hospital, examination center, or the like, a plurality of such automatic analysis devices and sample pre-processing devices are generally disposed and used in a clinical laboratory.

When a sample is analyzed by such an automatic analysis device, if a reagent runs out during the analysis, the analysis may be delayed and the diagnosis of a doctor may be delayed unless dealt with immediately. Patent Literature 1 discloses a technique of predicting an operation status of an automatic analysis device on the current day based on a past analysis history, and displaying a timing of replacing a reagent based on the predicted operation status and a remaining amount of the reagent on a screen.

Patent Literature 2 discloses a technique of measuring a quality management sample performed from a viewpoint of quality management of an analysis result, in which when the quality management sample is measured after replacement of a reagent bottle, analysis of a general sample cannot be performed during that time, and thus the quality management sample is held in a buffer, and when an amount of reagent is equal to or less than a set value, recommendation of measurement of the quality management sample for a standby reagent bottle is displayed on a screen or executed automatically.

Citation List

Patent Literature

Patent Literature 1: JP-A-2019-45446
Patent Literature 2: JP-A-2004-271265

Summary of Invention

Technical Problem

In order to analyze a sample with an automatic analysis device, it is necessary to perform calibration for obtaining a calibration curve for calculating a concentration of the sample at the time of sample replacement or periodically, and QC measurement for evaluating whether a reagent is measured correctly. In the present application, hereinafter, processing for optimizing analysis by a reagent such as the calibration and the QC measurement is collectively referred to as calibration/quality management processing, and samples used in the calibration/quality management processing (for example, a calibrator used for the calibration or a QC sample used for the QC measurement) are collectively referred to as a calibration/quality management sample.

As disclosed in Patent Literature 2, since reagent replacement associates with the calibration/quality management processing, it is necessary to prevent time loss for the calibration/quality management processing. Patent Literatures 1 and 2 are both techniques for avoiding stagnation of analysis associated with the reagent replacement, and are techniques in units of automatic analysis device. Therefore, the automatic analysis device in Patent Literature 2 requires a buffer for holding the quality management sample.

On the other hand, in a clinical laboratory having a relatively large scale, a plurality of automatic analysis devices may be disposed, and the same reagent may be used in the plurality of automatic analysis devices. Since the reagent and the calibration/quality management sample each have an expiration date and affect an operation cost of the clinical laboratory, it is desirable to use the reagent and the calibration/quality management sample optimally in the entire clinical laboratory.

In the invention, the reagent replacement and the calibration/quality management processing for the plurality of automatic analysis devices are managed in units of clinical laboratory. At this time, by requesting a laboratory technician to prepare in consideration of a time required for preparation, it is possible to increase an operation rate of the automatic analysis device disposed in the clinical laboratory. Alternatively, the operation cost of the clinical laboratory can be optimized by appropriately selecting the reagent or the calibration/quality management sample of the clinical laboratory. Further, by increasing the operation rate of the automatic analysis device in units of clinical laboratory, a mechanism mounted on each automatic analysis device can be simplified, and the cost of the clinical laboratory can be reduced.

Solution to Problem

A management device according to an embodiment of the invention is a management device configured to manage a plurality of analysis devices in a clinical laboratory provided with the plurality of analysis devices that analyze a sample and a cabinet that stores a reagent used for measurement by the plurality of analysis devices or a calibration/quality management sample used for calibration/quality management processing of the reagent, the management device includes: a storage device configured to store a reagent information management table that manages a remaining amount of each of in-measurement reagents that are respectively installed on the plurality of analysis devices so as to be used for measurement, a reagent replacement preparation time management table that registers a first preparation time required for preparation for replacing the in-measurement reagent with a storage reagent stored in the cabinet and a calibration/quality management processing preparation time management table that registers a second preparation time required for preparation for calibration/quality management processing of the storage reagent; a reagent replacement preparation timing detection unit configured to detect a timing to start reagent replacement preparation, based on the remaining amount of the in-measurement reagent managed in the reagent information management table and the first preparation time registered in the reagent replacement preparation time management table; a calibration/quality management processing preparation timing detection unit configured to detect a timing to start calibration/quality management processing preparation, based on the remaining amount of the in-measurement reagent managed in the reagent information management table and the second preparation time registered in the calibration/quality management processing preparation time management table; and a notification person-in-charge determination unit configured to notify one of laboratory technicians working in the clinical laboratory of a request for the reagent replacement preparation or the calibration/quality management processing preparation upon receiving detection of a timing by the reagent replacement preparation timing detection unit or the calibration/quality management processing preparation timing detection unit.

Advantageous Effects of Invention

According to the invention, it is possible to increase an operation rate of an automatic analysis device disposed in a clinical laboratory.

Other problems and novel features will become apparent from the description of the present description and the accompanying drawings.

Brief Description of Drawings

[FIG. 1] FIG. 1 shows an example of a clinical laboratory.
[FIG. 2] FIG. 2 is a block diagram showing a schematic configuration of an automatic analysis system.
[FIG. 3] FIG. 3 shows an example of a reagent information management table.
[FIG. 4] FIG. 4 shows an example of a reagent replacement preparation time management table.
[FIG. 5] FIG. 5 shows an example of a calibration preparation time management table.
[FIG. 6] FIG. 6 shows an example of a QC measurement preparation time management table.
[FIG. 7] FIG. 7 shows an example of a person-in-charge schedule management table.
[FIG. 8] FIG. 8 shows an example of a person-in-charge position information management table.
[FIG. 9] FIG. 9 shows an example of a clinical laboratory map.
[FIG. 10] FIG. 10 shows an example of a reagent replacement preparation notification screen.
[FIG. 11] FIG. 11 shows an example of a calibration preparation notification screen.

[FIG. 12] FIG. 12 shows an example of a QC measurement preparation notification screen.

[FIG. 13] FIG. 13 is a flowchart showing a notification processing logic.

[FIG. 14] FIG. 14 shows an example of a reagent storage information management table.

[FIG. 15] FIG. 15 shows an example of a calibrator storage information management table.

[FIG. 16] FIG. 16 shows an example of a QC sample storage information management table.

[FIG. 17] FIG. 17 shows an example of a reagent replacement preparation notification screen.

[FIG. 18] FIG. 18 is a flowchart showing a recommended target determination logic.

[FIG. 19] FIG. 19 shows an example of a person-in-charge skill level management table.

[FIG. 20] FIG. 20 is a flowchart showing a notification processing logic.

Description of Embodiments

First Embodiment

In a first embodiment, in a situation where the number of times of remaining measurement of a reagent (in-measurement reagent) in an analysis device in a clinical laboratory is small and a reagent replacement time is close, a management device detects a reagent replacement timing at which reagent replacement should be started, a calibration preparation timing at which preparation for performing calibration on the replaced reagent (storage reagent) should be started, or a QC measurement preparation timing at which preparation for performing QC measurement on the replaced reagent (storage reagent) should be started, and notifies a laboratory technician of the reagent replacement timing, the calibration preparation timing, or the QC measurement preparation timing as a task. At this time, tasks are requested in an order of appropriate laboratory technicians in consideration of the tasks and locations currently engaged by the laboratory technicians working in the clinical laboratory.

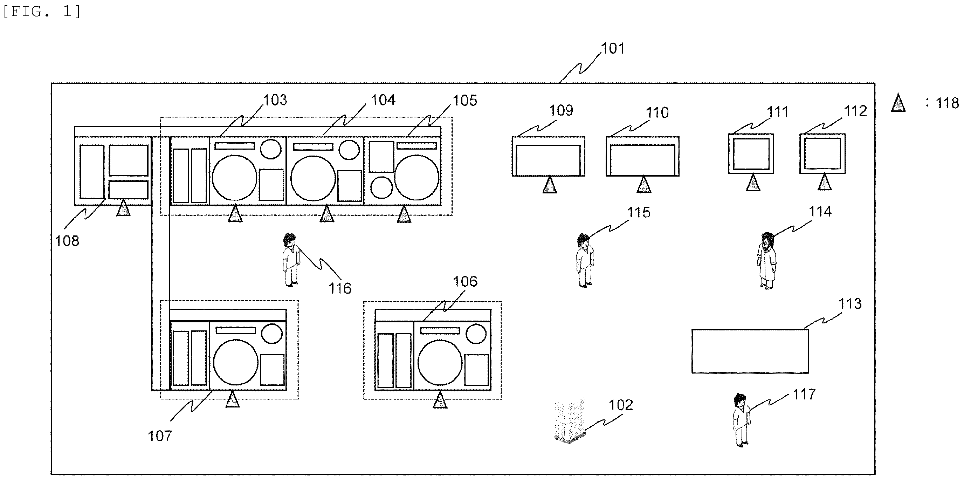

FIG. 1 shows an example of a clinical laboratory. Various devices, cabinets, work tables, and the like are disposed in a clinical laboratory 101, which is a region where clinical examinations are performed. In the example, a management device 102, analysis devices 103, 104, 105, 106, and 107, a pre-processing device 108, reagent cabinets 109 and 110, a calibrator cabinet 111, a QC sample cabinet 112, and a work table 113 are disposed, and laboratory technicians 114, 115, 116, and 117 are present in the room. Although not shown in FIG. 1, it is assumed that a position information acquisition device for acquiring position information on the laboratory technician is also located in the clinical laboratory 101.

The management device 102 communicates with the analysis devices 103 to 107, the pre-processing device 108, and terminals of the laboratory technicians via a network, and manages these devices. The analysis devices 103 to 107 are devices for performing the clinical examination, and may be operated independently or may be operated by combining a plurality of analysis devices. In this example, the analysis devices 106 and 107 operate independently, and the analysis devices 103, 104, and 105 operate in combination. The pre-processing device 108 is a device that automatically performs pre-processing for performing the clinical examination, is connected to the analysis devices 103 to 105 and 107, and transports a sample after the pre-processing to the analysis device. The management device 102 manages alarms generated by the analysis devices 103 to 107 and the pre-processing device 108 and alarms generated by the management device 102 itself. The reagent cabinets 109 and 110 store reagents necessary for the analysis device to perform the analysis. The calibrator cabinet 111 and the QC sample cabinet 112 store calibrators and QC samples for performing calibration and QC measurement in the analysis device. The laboratory technicians 114 to 117 are persons in charge who prepare the clinical examination in the clinical laboratory 101, operate the analysis devices, the pre-processing device, and the like, and each of the laboratory technicians 114 to 117 carries one or more terminals connected to the management device 102 via a network. After receiving a reagent replacement preparation notification, a calibration preparation notification, and a QC measurement preparation notification, the laboratory technicians 114 to 117 move to the front sides of the cabinets in order to take out the reagent, the calibrator, and the QC sample for the reagent replacement, the calibration preparation, and the QC measurement preparation. The front sides of the devices or the cabinets in the clinical laboratory 101 where the laboratory technicians work are indicated by triangle marks 118.

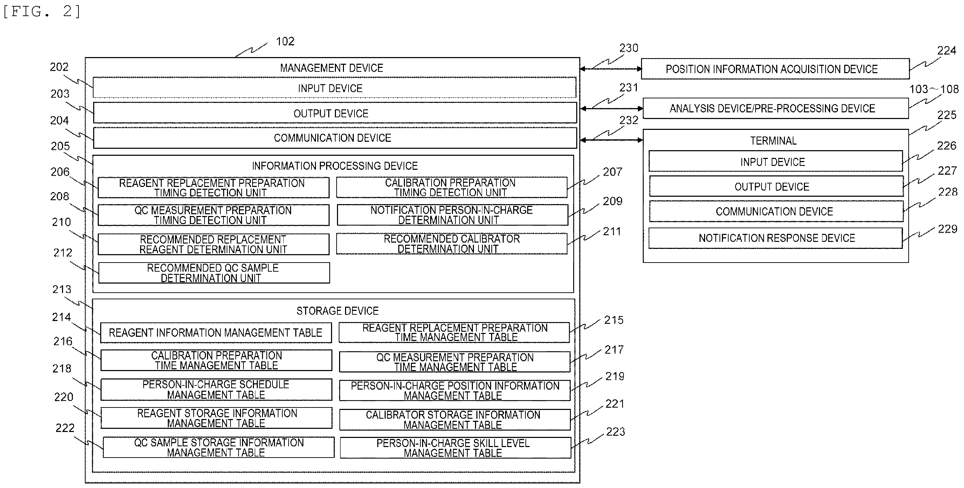

FIG. 2 is a block diagram showing a schematic configuration of an automatic analysis system. A terminal 225 is a terminal carried by the laboratory technicians 114 to 117 in FIG. 1. The management device 102 is communicably connected to a position information acquisition device 224 via a network 230, to the analysis devices 103 to 107 via a network 231, to the pre-processing device 108 via the network 231, and to the terminal 225 via a network 232.

The management device 102 includes an input device 202, an output device 203, a communication device 204, an information processing device 205, and a storage device 213. The input device 202 is, for example, a pointing device such as a keyboard or a mouse, and is a device for inputting an instruction from a laboratory technician or an administrator. It is also possible to use a screen touch panel or the like as the output device 203. The output device 203 is a monitor or a printer that displays input information from the input device 202 and other result information. The communication device 204 is a communication device for the management device 102 to communicate with the position information acquisition device 224, the analysis devices/pre-processing device 103 to 108, and the terminal 225 via the networks, and a wired/wireless local area network, a wired/wireless global area network, a mobile phone line network, or a combination thereof can be used.

The information processing device 205 is a computer for executing various applications of the management device 102. The information processing device 205 includes a reagent replacement preparation timing detection unit 206 which is a logic for detecting a timing at which the reagent is to be replaced, a calibration preparation timing detection unit 207 which is a logic for detecting a timing at which the calibration preparation is to be started, a QC measurement preparation timing detection unit 208 which is a logic for detecting a timing at which the QC measurement preparation is to be started, a notification person-in-charge determination unit 209 which is a logic for determining a person in charge of task notification, a recommended replacement reagent determination unit 210 which is a logic for determining a recommended replacement reagent, a recommended calibrator determination unit 211 which is a logic for determining a recommended calibrator, and a recommended QC sample determination unit 212 which is a logic for determining a recommended QC sample. Details thereof will be described later.

The storage device 213 is a storage device that stores programs, data, and the like executed by the information processing device 205, and can use, for example, a nonvolatile memory such as an EEPROM or a flash memory, an HDD, or an SSD. The storage device 213 is mounted on the analysis devices 103 to 107, and includes a reagent information management table 214 for managing information on reagents used for performing the clinical examination, a reagent replacement preparation time management table 215 for managing a time required for the reagent replacement preparation for each reagent, a calibration preparation time management table 216 for managing a time required for the calibration preparation for each reagent, a QC measurement preparation time management table 217 for managing a time required for the QC measurement preparation for each reagent, a person-in-charge schedule management table 218 for managing tasks of each laboratory technician, a person-in-charge position information management table 219 for managing position information on each laboratory technician in the clinical laboratory 101 acquired by the position information acquisition device 224, a reagent storage information management table 220 for managing stock information on the reagents, a calibrator storage information management table 221 for managing stock information on the calibrators, a QC sample storage information management table 222 for managing stock information on the QC samples, and a person-in-charge skill level management table 223 for managing a skill level of each laboratory technician. Details thereof will be described later.

When various applications of the management device 102 are implemented by executing programs by the information processing device 205, programs corresponding to the logics executed by the information processing device 205 are stored in the storage device 213, and functions of the detection units 206 to 208 or the determination units 209 to 212 are implemented by the information processing device 205 reading and executing the programs.

For example, the position information acquisition device 224 may grasp the position information by using the terminal 225 carried by the laboratory technician, or may grasp the position information on the laboratory technician by image analysis of a surveillance camera image installed in the clinical laboratory 101. For example, global positioning system (GPS), Beacon, ultra wide band (UWB), Wi-Fi positioning, sonic positioning, dead reckoning, indoor messaging system (IMES), or a combination thereof can be used.

The laboratory technician browses, by using the terminal 225, the notification sent from the management device 102, responds to processing, and responds to completion of the processing. An input device 226, an output device 227, and a communication device 228 in the terminal 225 have the same functions as those of the management device 102. A notification response device 229 has a function of responding to the notification from the management device 102. The terminal 225 is preferably a mobile terminal or a wearable terminal carried by the laboratory technician, and a notebook or desktop PC or the like may be used.

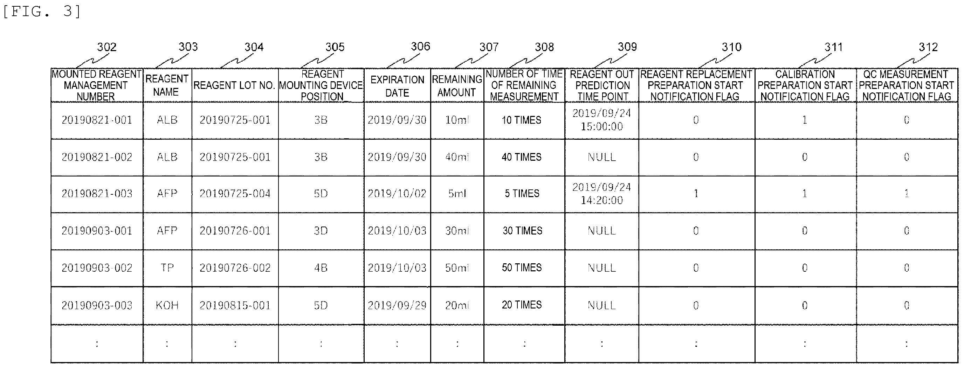

FIG. 3 shows the reagent information management table 214. The reagents mounted on the analysis devices 103 to 107 in the clinical laboratory 101 connected to the management device 102 via the network are all managed by the reagent information management table 214. When the reagent is mounted on the analysis device, the reagent is registered in the reagent information management table 214.

Column 302 is a mounted reagent management number, and is a number for identifying a reagent mounted on the analysis device. Column 303 is a reagent name. Column 304 is a reagent lot No. (reagent lot number), and the reagent lot No. is given when the reagent is manufactured by a reagent manufacturer. At the time of the reagent replacement, when the reagents before and after the replacement have the same reagent lot No., a calibration result can be taken over. That is, when the reagent to be replaced has the same reagent lot No., it is not necessary to perform the calibration at the time of the reagent replacement. Column 305 is a reagent mounting device position, and represents a position of the device on which the reagent is mounted. In this example, identification numbers defined in a clinical laboratory map to be described later are displayed. Column 306 is an expiration date of the reagent. A reagent that has passed the expiration date needs to be replaced. Column 307 is a remaining amount of the reagent. The analysis device may measure the remaining amount or may calculate the remaining amount based on the number of clinical examinations in the analysis device. Column 308 is the number of time of remaining measurement of the reagent, and can be calculated based on the remaining amount of the reagent in column 307. Column 309 is a reagent out prediction time point, and stores a time point predicted as a reagent out timing. The reagent out prediction time point can be predicted from the remaining amount of the reagent and an analysis frequency of the analysis device on the current day. For example, the number of time of remaining measurement in column 308 can be calculated by dividing the amount of reagent used per measurement from the remaining amount of reagent in column 307, and a reagent out time point in column 309 can be predicted from the analysis frequency of the analysis device on the current day. In the prediction of the reagent out time point, prediction quality is lowered when the remaining amount of the reagent is sufficient, and therefore, when the number of time of remaining measurement of the reagent exceeds 10, the reagent out prediction time point in column 309 is set to NULL.

Column 310 is a reagent replacement preparation start notification flag, in which zero is registered as an initial value, and the value becomes 1 at a timing when the reagent replacement preparation is to be started. Column 311 is a calibration preparation start notification flag, in which zero is registered as an initial value, and the value is set to 1 at a timing at which the calibration preparation is to be started. Column 312 is a QC measurement preparation start notification flag, in which zero is registered as an initial value, and the value becomes 1 at a timing when the QC measurement preparation is to be started. Hereinafter, a logic for setting these flags to 1 will be described. These flags are updated by the reagent replacement preparation timing detection unit 206, the calibration preparation timing detection unit 207, and the QC measurement preparation timing detection unit 208, respectively.

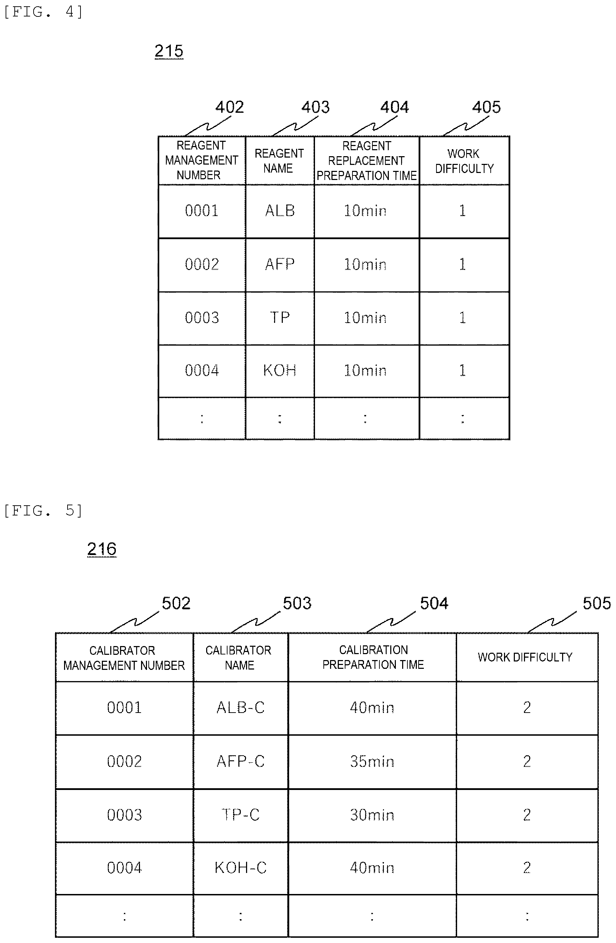

FIG. 4 shows the reagent replacement preparation time management table 215. The reagent replacement is not to simply take out the reagents from the reagent cabinets 109 and 110 and put the reagents into the device, and may be performed by putting the reagents into the device immediately after taking out from the cabinets, or by putting the reagents into the device after waiting until the reagents reach a room temperature, which results in a different preparation time for each reagent. Therefore, a time required for the reagent replacement preparation for each reagent is registered in the reagent replacement preparation time management table 215. The time required for the reagent replacement preparation may be registered in advance by a reagent manufacturer or may be registered by the administrator of the clinical laboratory. A reagent replacement preparation time may be calculated and updated in the clinical laboratory based on an actual result of the time required for the laboratory technician to prepare the reagent. Column 402 is a reagent management number, and is a management number associated with a reagent name in column 403 on a one-to-one basis. Column 404 is a reagent replacement preparation time, and indicates the time required for the laboratory technician to prepare for the reagent replacement. The reagent replacement preparation time in column 404 is used to set the reagent replacement preparation start notification flag (column 310) in the reagent information management table 214 (see FIG. 3) to 1. The reagent replacement preparation timing detection unit 206 (management device 102) updates the reagent replacement preparation start notification flag from 0 to 1 at a time point when a current time point goes back from the reagent out prediction time point (column 309) by the reagent replacement preparation time (column 404). For example, when the reagent out prediction time point is 14:20 and the reagent replacement preparation time is 10 minutes, the reagent replacement preparation start notification flag is updated to 1 when the current time point is 14:10. Column 405 is work difficulty, and indicates difficulty of the reagent replacement preparation for each reagent in five stages. The work difficulty in column 405 being 1 indicates the lowest work difficulty, and the work difficulty being 5 indicates a high work difficulty.

FIG. 5 shows the calibration preparation time management table 216. When performing the calibration, a worker takes out a calibrator from the calibrator cabinet 111, thaws and stirs the calibrator, and then puts the calibrator into the device. The calibrator is a sample having a known concentration, and is used for creating a calibration curve. The calibrator may also be used immediately after being taken out from the cabinet, or may be used after waiting until the calibrator reaches the room temperature. Further, since a stirring time is different for each calibrator, a preparation time is different for each calibrator. In the calibration preparation time management table 216, a time required for the calibration preparation is registered for each calibrator. Similar to the reagent, the time required for the calibration preparation may be registered in advance by the manufacturer or may be registered by the administrator of the clinical laboratory. A calibration preparation time may be updated based on an actual result of the time required for the laboratory technician to prepare for the calibration. Column 502 is a calibrator management number, and column 503 is a calibrator name, which are on a one-to-one basis, similar to the reagent. Column 504 is a calibration preparation time, and indicates the time required for the laboratory technician to prepare for the calibration. The calibration preparation time in column 504 is used to set the calibration preparation start notification flag (column 311) in the reagent information management table 214 (see FIG. 3) to 1. The calibration preparation timing detection unit 207 (management device 102) updates the calibration preparation start notification flag from 0 to 1 at a time point when the current time point goes back from the reagent out prediction time point (column 309) by the calibration preparation time (column 504). For example, when the reagent out prediction time point is 14:20 and the calibration preparation time is 40 minutes, the calibration preparation start notification flag is updated to 1 when the current time point becomes 13:40. Column 505 is work difficulty, and indicates difficulty of the calibration preparation for each calibrator in five stages. The work difficulty in column 505 being 1 indicates the lowest work difficulty, and the work difficulty being 5 indicates a high work difficulty.

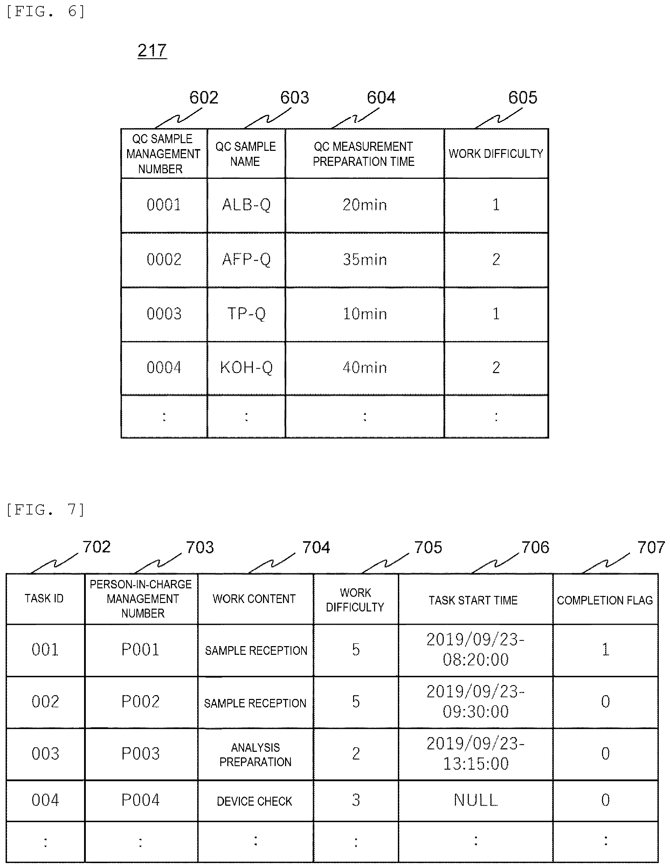

FIG. 6 shows the QC measurement preparation time management table 217. When performing the QC measurement, the worker takes out a QC sample from the QC sample cabinet 112, thaws and stirs the QC sample, and then puts the QC sample into the device. The QC sample is a sample for confirming that the quality of the analysis device is maintained, and is used in units of several months or year. The QC sample may also be used immediately after being taken out from the cabinet, or may be used after waiting until the QC sample reaches the room temperature. Further, since a stirring time is different depending on the QC sample, the preparation time is different depending on the QC sample. In the QC measurement preparation time management table 217, a time required for the QC measurement preparation is registered for each QC sample. Similar to the reagent, the time required for the QC measurement preparation may be registered in advance by the manufacturer or may be registered by the administrator of the clinical laboratory. A QC measurement preparation time may be updated based on an actual result of the time required for the laboratory technician to prepare for the QC measurement. Column 602 is a QC sample management number, and column 603 is a QC sample name, which are on a one-to-one basis, similar to the reagent. Column 604 is a QC measurement preparation time, and indicates the time required for the laboratory technician to prepare for the QC measurement. The QC measurement preparation time in column 604 is used to set the QC measurement preparation start notification flag (column 312) in the reagent information management table 214 (see FIG. 3) to 1. The QC measurement preparation timing detection unit 208 (management device 102) updates the QC measurement preparation start notification flag from 0 to 1 at a time point when a current time point goes back from the reagent out prediction time point (column 309) by the QC measurement preparation time (column 604). For example, when the reagent out prediction time point is 14:20 and the QC measurement preparation time is 20 minutes, the QC measurement preparation start notification flag is updated to 1 when the current time point becomes 14:00. Column 605 is work difficulty, and indicates difficulty of the QC measurement preparation for each QC sample in five stages. The work difficulty in column 605 being 1 indicates the lowest work difficulty, and the work difficulty being 5 indicates a high work difficulty.

FIG. 7 shows the person-in-charge schedule management table 218. The person-in-charge schedule management table 218 manages the tasks performed by the laboratory technicians and the difficulty of the tasks. Column 702 is a task ID and an identification number of the task performed by the laboratory technician. Column 703 is a person-in-charge management number and is an identification number of the laboratory technician who performs the task. Column 704 is a work content, and is a content of the task. In addition to the tasks listed in the drawing, for example, the reagent replacement preparation, the calibration preparation, and the QC measurement preparation are also tasks registered in column 704. Column 705 is work difficulty. As for the work difficulty, it is assumed that a task that takes time to complete the work has higher difficulty, and a task that can be completed in a short time has lower difficulty. Here, evaluation is performed in five stages from 5 to 1, and the greater the value of the work difficulty is, the higher the difficulty is. Column 706 is a task start time, and registers a time at which the laboratory technician starts the task. The management device 102 detects the task start time by the notification from the terminal carried by the laboratory technician or other methods, and updates the task start time in column 706. An initial value of the task start time in column 706 is NULL. Column 707 is a completion flag, and manages whether the laboratory technician has completed the task. In the completion flag in column 707, 0 representing that the task is not completed is registered as an initial value, and 1 representing that the task is completed is registered after the task is completed. The management device 102 detects the completion of the task by the notification from the terminal carried by the laboratory technician or other methods, and updates the completion flag in column 707.

As will be described later, the reagent replacement preparation, the calibration preparation, and the QC measurement preparation are notified, and when the laboratory technician responds an intention to process, each is registered as a new task in the person-in-charge schedule management table 218. The work difficulty registered in the reagent replacement preparation time management table 215, the calibration preparation time management table 216, and the QC measurement preparation time management table 217 is registered as the work difficulty at this time.

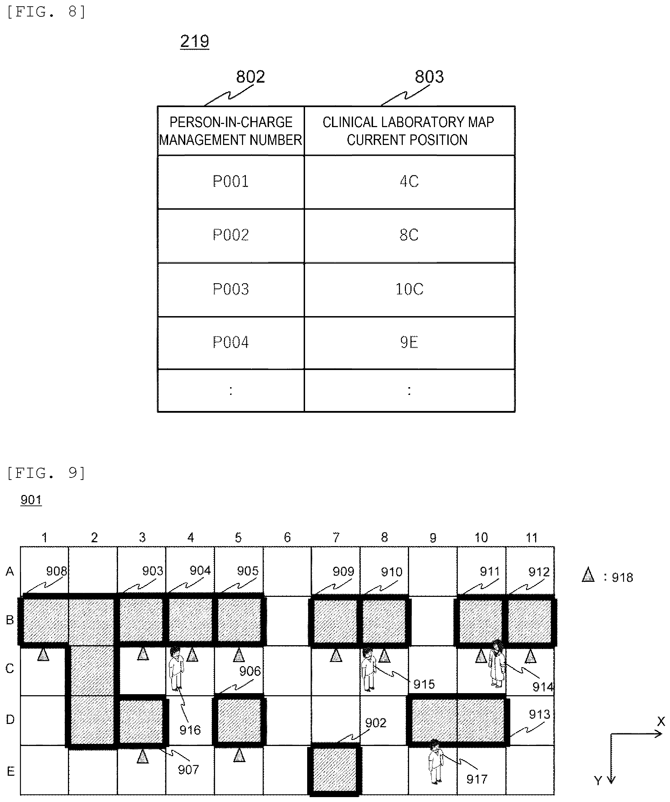

FIG. 8 shows the person-in-charge position information management table 219. The position information on the laboratory technician in the clinical laboratory 101 is measured in real time or at regular time intervals, and is registered in the person-in-charge position information management table 219. Column 802 is a person-in-charge management number, and is a number for specifying the laboratory technician. Column 803 is a clinical laboratory map current position, and is position information on the laboratory technician determined by the clinical laboratory map to be described later.

FIG. 9 is a clinical laboratory map 901, in which positions in the clinical laboratory 101 are specified by the identification numbers. The map in FIG. 9 corresponds to the clinical laboratory 101 in FIG. 1. In the clinical laboratory map 901, the management device 102 is disposed in a region 902, the analysis devices 103 to 107 are disposed in regions 903 to 907, respectively, the pre-processing device 108 is disposed in a region 908, the reagent cabinets 109 and 110 are disposed in regions 909 and 910, respectively, the calibrator cabinet 111 is disposed in a region 911, the QC sample cabinet 112 is disposed in a region 912, and the work table 113 is disposed in a region 913. The regions in which these devices/storage cabinets/work table are disposed are shaded to be distinguished from other regions. Further, current locations of the laboratory technicians 114 to 117 are displayed as laboratory technicians 914 to 917, respectively.

In the clinical laboratory map 901, the clinical laboratory is divided into blocks, and the identification number is defined by an X coordinate and a Y coordinate of the block. The X coordinate is defined by integers from 1 to 11 in order from a left side to a right side, and the Y coordinate is defined by letters from A to E from the top to the bottom. For example, the position of the laboratory technician 917 is represented as 9E since the X coordinate is 9 and the Y coordinate is E. As described above, the shaded blocks in the clinical laboratory map 901 are blocks in which the devices/cabinets/work table are disposed, and thus the laboratory technicians cannot move on the shaded blocks. Triangular marks 918 represent front sides of the devices or the cabinets, and when the laboratory technician moves to the device or the cabinet for working, the laboratory technician moves to a block having the triangular mark 918. For example, when the laboratory technician 916 moves to the pre-processing device 908, a path order is 4C, 4D, 4E, 3E, 2E, 1E, 1D, and 1C, and a distance at that time is 7 because movement of the laboratory technician is 7 blocks.

On the other hand, with respect to a management device or work table for which the front side 918 is not designated, calculation is performed assuming that the laboratory technician moves to the corresponding block. When a plurality of shaded blocks are connected, the calculation is performed assuming movement to a position of a block at which the numerical value of the X coordinate is the smallest and the letter of the Y coordinate is closest to A.

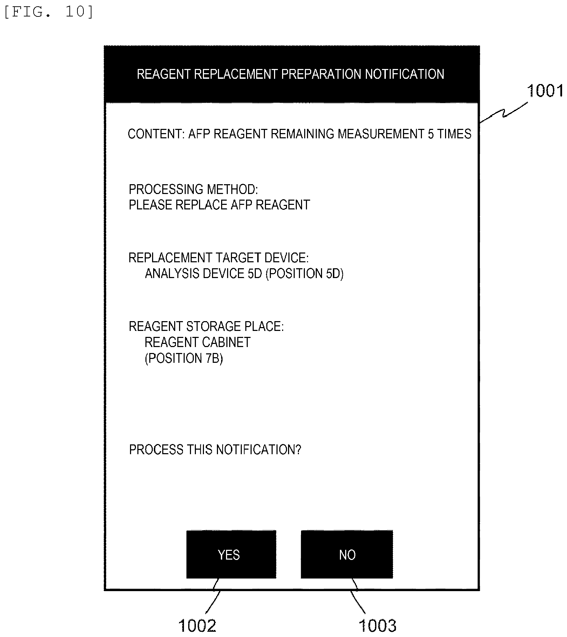

FIG. 10 shows a reagent replacement preparation notification screen 1001. The laboratory technician confirms a reagent replacement preparation request through the reagent replacement preparation notification screen 1001 displayed on the terminal 225, and responds an intention to be the person in charge of the reagent replacement. A button 1002 is a "YES" button, and is a button for the laboratory technician to respond the intention to be the person in charge of the notification. A button 1003 is a "NO" button, and is a button for the laboratory technician to respond the intention of not being able to be the person in charge of the notification.

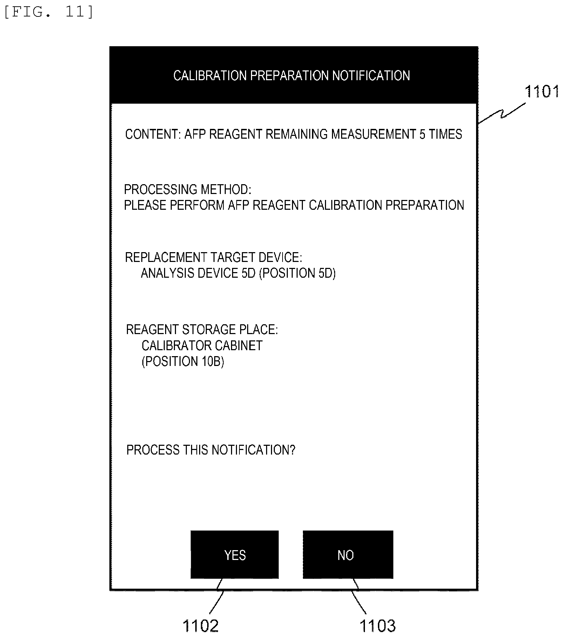

FIG. 11 shows a calibration preparation notification screen 1101. As in FIG. 10, the laboratory technician responds the intention to be the person in charge of the notified calibration by using buttons 1102 and 1103.

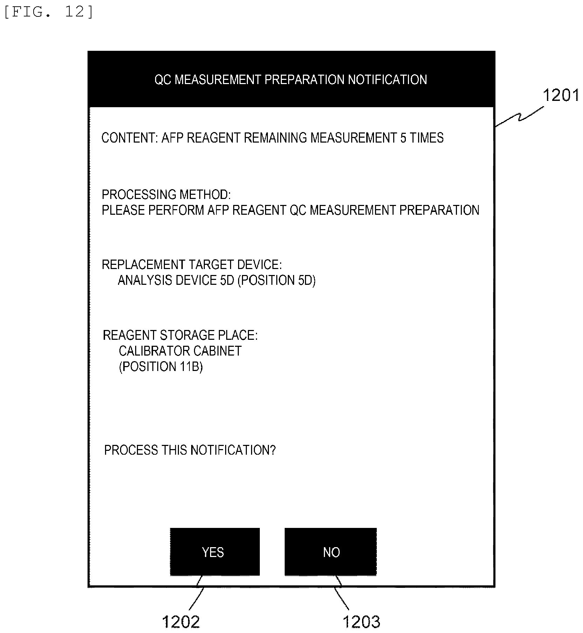

FIG. 12 shows a QC measurement preparation notification screen 1201. As in FIG. 10 or FIG. 11, the laboratory technician responses the intention to be the person in charge of the notified QC measurement by using buttons 1202 and 1203.

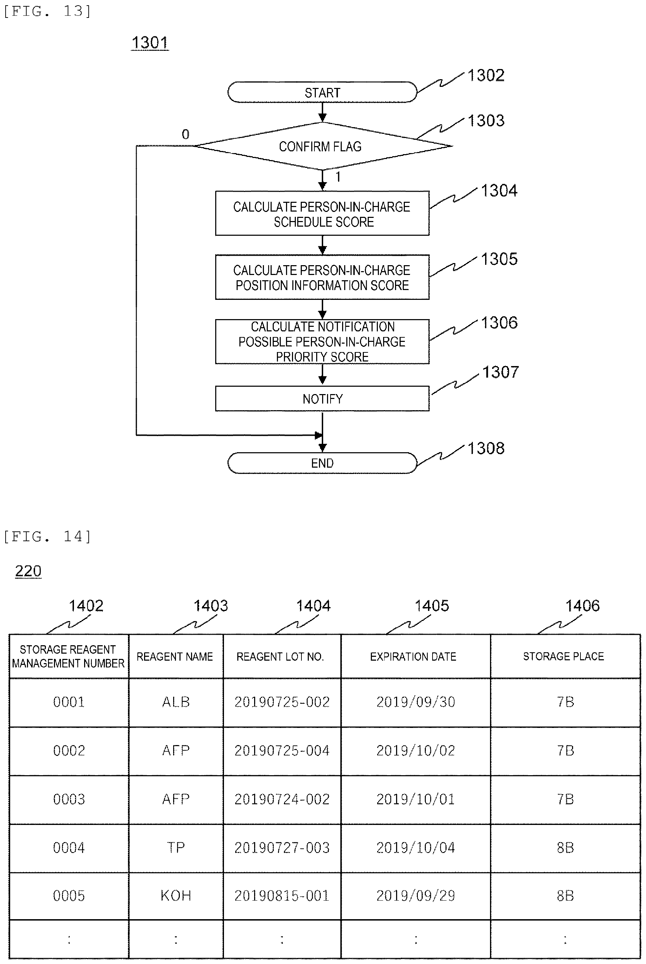

FIG. 13 shows a notification processing logic 1301 executed by the notification person-in-charge determination unit 209 (see FIG. 2). The management device 102 executes the processing of the notification processing logic 1301 on the reagents registered in the reagent information management table 214 (see FIG. 3). The notification processing logic 1301 is commonly used when the reagent replacement preparation notification, the calibration preparation notification, and the QC measurement preparation notification are executed, and a flag to be confirmed in flag confirmation 1303 and a position information reference point in person-in-charge position information score calculation 1305 are different depending on a notification content. Hereinafter, the processing of determining the person in charge to be notified of a reagent replacement preparation task will be mainly described.

The flag confirmation 1303 is processing of confirming whether the management device 102 needs any one of the reagent replacement preparation notification, the calibration preparation notification, and the QC measurement preparation notification. Therefore, the management device 102 confirms the reagent replacement preparation start notification flag (column 310), the calibration preparation start notification flag (column 311), and the QC measurement preparation start notification flag (column 312) in the reagent information management table 214.

When the reagent replacement preparation start notification flag (column 310) is 1 representing that the reagent replacement preparation notification is necessary, processing of person-in-charge schedule score calculation 1304 is executed. On the other hand, when the reagent replacement preparation start notification flag (column 310) is 0 representing that the reagent replacement preparation notification is unnecessary, if there is no other record in which the reagent replacement preparation start notification flag is 1, the processing proceeds to the end 1308.

The person-in-charge schedule score calculation 1304 refers to the person-in-charge schedule management table 218 (see FIG. 7) and acquires the difficulty of currently working tasks for all the laboratory technicians in the clinical laboratory 101. In the person-in-charge schedule score calculation 1304, for the currently working tasks, the task start time (column 706) is registered with a value other than the initial value NULL, and the completion flag (column 707) is registered with 0 indicating that the task is not completed. In the person-in-charge schedule score calculation 1304, the work difficulty is acquired from column 705. When the work difficulty cannot be acquired, it is determined that there is no working task, and the work difficulty is set to 1. A person-in-charge schedule score is set as 5 points, 4 points, 3 points, 2 points, and 1 point based on the work difficulty evaluated in five stages including 5, 4, 3, 2, and 1, and the higher the score is, the higher the work difficulty is. As a result of the person-in-charge schedule score calculation 1304, a person-in-charge schedule score s for each laboratory technician in the clinical laboratory 101 is output.

The person-in-charge position information score calculation 1305 calculates a person-in-charge position information score based on the position information on all the laboratory technicians currently in the clinical laboratory 101. The position information on the laboratory technicians is acquired from the position information acquisition device 224 as the identification number in the clinical laboratory map 901. Next, in the case of the reagent replacement preparation notification, the position information on the reagent cabinets 109 and 110 is acquired as a position information reference point. In the case of the calibration preparation notification, the position information on the calibrator cabinet 111 is acquired as a position information reference point, and in the case of the QC measurement preparation notification, the position information on the QC sample cabinet 112 is acquired as a position information reference point. In order to execute the task, the person in charge moves to the position information reference point and then moves to the analysis device or the work table, whereas the movement after the position information reference point is the same regardless of the person in charge. Therefore, in the person-in-charge position information score calculation 1305, the shortest distance until each laboratory technician reaches the position information reference point is calculated based on the acquired position information reference point and the position information on the laboratory technician. Specifically, the person-in-charge position information score is calculated as the shortest distance for reaching the front side 918 of the cabinet which is the position information reference point based on the clinical laboratory map 901. As a result, a person-in-charge position information score p for each laboratory technician is output. When there are a plurality of position information reference points, the shortest distance may be calculated for each of the plurality of position information reference points, and an average value thereof may be set as the person-in-charge position information score p.

Notification possible person-in-charge priority score calculation 1306 is processing of performing notification priority score calculation for determining a priority to be notified to the laboratory technicians in the clinical laboratory 101 by using the person-in-charge schedule score and the person-in-charge position information score calculated in the person-in-charge schedule score calculation 1304 and the person-in-charge position information score calculation 1305 as input. For example, a notification priority score Pr is given by the following (Formula 1).

$$Pr = m_1 \cdot s + m_2 \cdot p \quad \text{(Formula 1)}$$

Here, $m_1$ is a weight for the person-in-charge schedule score s represented by a positive constant, and $m_2$ is a weight for the person-in-charge position information score p represented by a positive constant. The smaller the notification priority score Pr, the higher the priority, and the larger the notification priority score Pr, the lower the priority. As a result, the notification priority score Pr is output to all the laboratory technicians in the clinical laboratory 101.

Notification 1307 is processing of transmitting a notification to the terminal 225 carried by the laboratory technician having the highest priority based on a result in the notification possible person-in-charge priority score calculation 1306 and acquiring a response from the laboratory technician at a transmission target. When a response of not being able to be the person in charge is received from the laboratory technician, the laboratory technician is excluded until a certain period of time elapses, the person-in-charge schedule score calculation 1304, the person-in-charge position information score calculation 1305, and the notification possible person-in-charge priority score calculation 1306 are performed again, and a notification is transmitted to the next notification target until the person in charge is determined. When the response of not being able to be the person in charge is received from all the laboratory technicians in the clinical laboratory 101, a notification is transmitted to the administrator who collects the tasks of the laboratory technicians in the clinical laboratory 101.

A specific example of processing in the clinical laboratory according to the first embodiment will be described. Here, a case where a remaining amount of an AFP reagent is small in the analysis device 106 (reagent mounting device position 5D) and the reagent replacement is necessary will be described.

After the reagent replacement preparation timing detection unit 206 updates the reagent replacement preparation start notification flag, the notification person-in-charge determination unit 209 performs the processing of the flag confirmation 1303. The reagent information management table 214 at this time is shown in FIG. 3. That is, in a record of the mounted reagent management number (column 302) "20190821-003", the reagent replacement preparation start notification flag (column 310) is 1. Therefore, the processing of the person-in-charge schedule score calculation 1304 is performed. The person-in-charge schedule management table 218 at this time is shown in FIG. 7. A laboratory technician P001 is in a state of completing the task he/she was doing and currently having no task to do, a laboratory technician P002 is in a state of working on a task having the work difficulty of 5, a laboratory technician P003 is in a state of working on a task having the work difficulty of 2, and a laboratory technician P004 is in a state of currently having no task to do. It is assumed that the laboratory technicians having the management numbers P001, P002, P004, and P005 are the laboratory technicians 114 to 117 in FIG. 1, respectively. Therefore, the person-in-charge schedule scores s of the laboratory technicians 114 to 117 are 1 point, 5 points, 2 points, and 1 point, respectively.

Subsequently, the notification person-in-charge determination unit 209 performs the processing of the person-in-charge position information score calculation 1305. Locations of the laboratory technicians at this time are as shown in the clinical laboratory map 901 in FIG. 9, and the position information on the laboratory technicians 114 to 117 is 10C, 8C, 4C, and 9E, respectively. Two reagent cabinets serve as the position information reference points and are disposed at 7B and 8B, respectively, and the front side 918 is set for each cabinet. Therefore, calculating a shortest distance from the current position of each laboratory technician to the reagent cabinets, the person-in-charge position information scores p of the laboratory technicians 114 to 117 are 2.5 points, 0.5 points, 3.5 points, and 3.5 points, respectively. Since two reagent cabinets are position information reference points, the shortest distance to each reagent cabinet is calculated, and an average thereof is set as the person-in-charge position information score p.

Subsequently, the notification person-in-charge determination unit 209 performs the notification possible person-in-charge priority score calculation 1306 to calculate the notification priority score Pr. At this time, if the notification priority score Pr is calculated using (Formula 1) in which the weight $m_1$ is 1 and the weight $m_2$ is 1, the notification priority scores Pr of the laboratory technicians 114 to 117 are 3.5 points, 5.5 points, 5.5 points, and 4.5 points, respectively.

As a result, the notification person-in-charge determination unit 209 transmits the reagent replacement preparation notification (see FIG. 10) to the terminal 225 carried by the laboratory technician 114 having the lowest score. After transmitting the notification, the notification person-in-charge determination unit 209 waits for a response from the laboratory technician 114 as to whether to be the person in charge of the reagent replacement. If the response is not obtained for a certain period of time or longer, the notification person-in-charge determination unit 209 determines that the laboratory technician 114 cannot be the person in charge, and performs the processing for determining the notification target again.

The processing for the reagent replacement preparation notification is described above, and a flow of the processing is the same for the calibration preparation notification and the QC measurement preparation notification.

Second Embodiment

In a second embodiment, in addition to the first embodiment, the management device specifies and presents a recommended target (replacement reagent, calibrator, QC sample) at the time of a task request to a laboratory technician.

In the present embodiment, it is assumed stocks of the reagents, the calibrators, and the QC samples are stored in the reagent cabinets 109 and 110, the calibrator cabinet 111, and the QC sample cabinet 112, respectively. Stock information on the reagents, the calibrators, and the QC samples is managed by the management device 102. The stock information on the reagents, the calibrators, and the QC samples is registered by reading the information on a barcode, a two-dimensional barcode, or an RFID attached to each container thereof. Specifically, when a person in charge of purchasing stores the reagents, the calibrators, and the QC samples in the reagent cabinets 109 and 110, the calibrator cabinet 111, and the QC sample cabinet 112, such stock information is registered in the management device 102. Instead of the person in charge of purchasing, a laboratory technician working in the clinical laboratory 101 may be registered.

The stock information on the reagents, the calibrators, and the QC samples is managed by the reagent storage information management table 220, the calibrator storage information management table 221, and the QC sample storage information management table 222 (see FIG. 2) stored in the storage device 213.

FIG. 14 shows the reagent storage information management table 220. Column 1402 is a storage reagent management number and is a management number for uniquely identifying the stock of the reagents in the clinical laboratory. Column 1403 is a reagent name, and is a name of the reagent notified to the person in charge at the time of the reagent replacement preparation notification. Column 1404 is a reagent lot No., and is a lot No. when the reagent is manufactured. Column 1405 is an expiration date, and only the reagents within the expiration date can be selected as the recommended replacement reagents. Column 1406 is a storage place, and represents a place where the reagent is stored by a code representing a position of the clinical laboratory map 901.

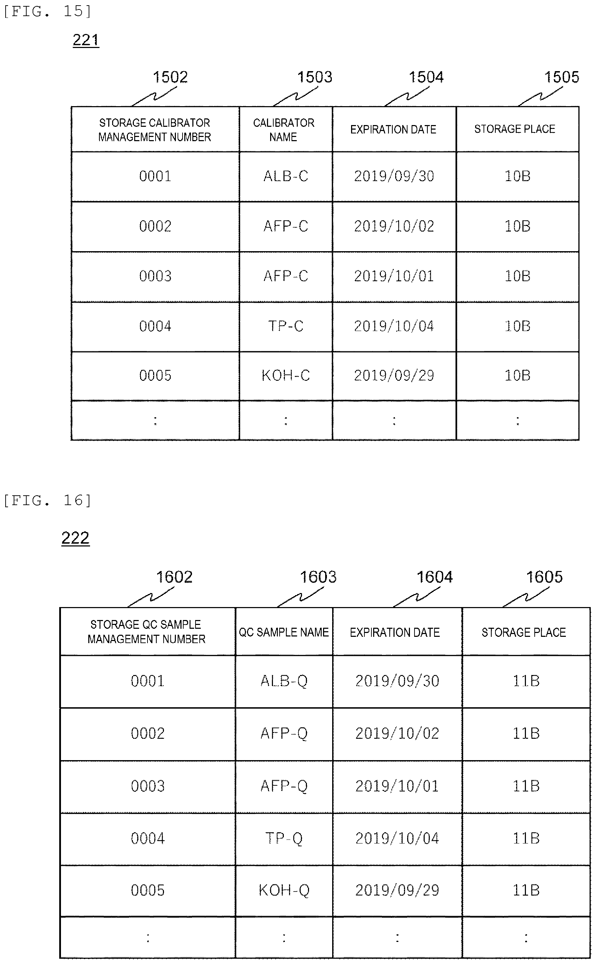

FIG. 15 shows the calibrator storage information management table 221. Column 1502 is a storage calibrator management number, and is a management number for uniquely identifying the stock of the calibrators in the clinical laboratory. Column 1503 is a calibrator name, and is a name of the calibrator notified to the person in charge at the time of the calibration preparation notification. Column 1504 is an expiration date, and only the calibrators within the expiration date can be selected as the recommended calibrators. Column 1505 is a storage place, and represents a place where the calibrator is stored by a code representing a position of the clinical laboratory map 901.

FIG. 16 shows the QC sample storage information management table 222. Column 1602 is a storage QC sample management number, and is a management number for uniquely identifying the stock of the QC samples in the clinical laboratory. Column 1603 is a QC sample name, and is a name of the QC sample notified to the person in charge at the time of the QC measurement preparation notification. Column 1604 is an expiration date, and only the QC samples within the expiration date can be selected as the recommended QC samples. Column 1605 is a storage place, and represents a place where the QC sample is stored by a code representing a position of the clinical laboratory map 901.

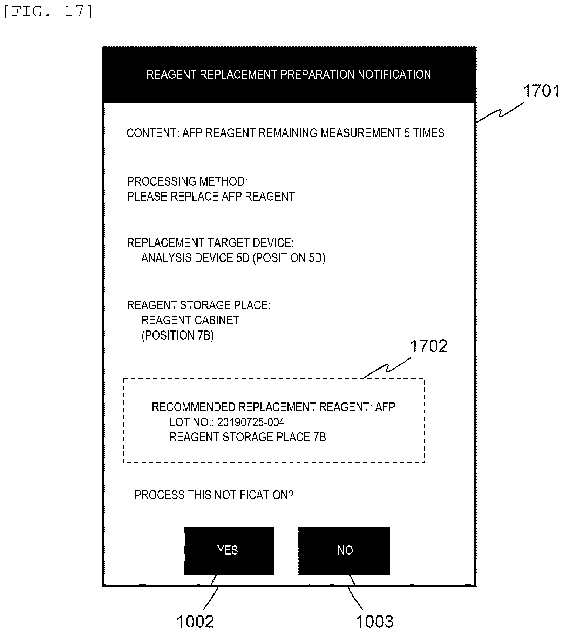

FIG. 17 shows a reagent replacement preparation notification screen 1701 according to the second embodiment. A difference from the reagent replacement preparation notification screen 1001 in the first embodiment is that a recommended replacement target reagent 1702 is displayed. In this example, a reagent having the same lot number as a reagent to be replaced is displayed as a recommended replacement target. The reagent having the same lot number has an effect of eliminating the need for calibration. Here, although a screen example of the reagent replacement preparation notification is described, a calibrator and a QC sample to be recommended can be similarly displayed on a calibration preparation notification screen and a QC measurement preparation notification screen. Specifically, by recommending a calibrator and a QC sample having a short period until the expiration date, it is possible to prevent the occurrence of a calibration/quality management sample out of the expiration date.

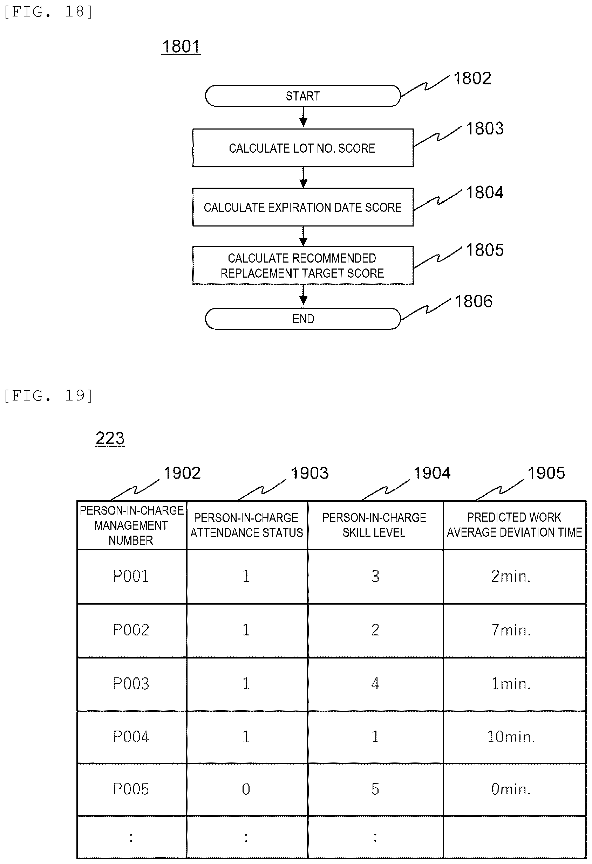

FIG. 18 shows a recommended target determination logic 1801, and is processing of scoring to determine whether the reagent to be replaced or the calibrator or the QC sample to be prepared is superior as a recommended target with respect to the stock of the reagents, the calibrators, or the QC samples in the clinical laboratory. If the automatic analysis system has a specification in which the calibration is not performed when the reagent to be replaced is the reagent having the same lot No., a burden on the laboratory technician can be reduced by replacing the reagent with the reagent having the same lot No. as much as possible. Therefore, in the case of the reagent replacement, two recommended conditions, that is, one having a close expiration date and one having the same lot No., are given. In the case of the calibrator or the QC sample, the recommended condition is one having a close expiration date. Each of the recommended replacement reagent determination unit 210, the recommended calibrator determination unit 211, and the recommended QC sample determination unit 212 performs processing according to the recommended target determination logic 1801.

Processing 1803 is processing of lot No. score calculation, and is executed when the replacement target is a reagent. That is, when the lot Nos. are the same in the reagent replacement preparation notification, 1 is output as a lot No. score l. On the other hand, at the time of the calibration preparation notification and the QC measurement preparation notification, 0 is always output as the lot No. score l.

Processing 1804 is processing of expiration date score calculation, and is processing of determining whether a recommended target is superior depending on whether the expiration date is close. For example, a difference between a current date and a due date is output as an expiration date score d. For example, when the current date is 2019/09/24 and the due date is 2019/09/28, 5 is output as the expiration date score d.

Processing 1805 is recommended target score calculation, and is processing of calculating a priority as the recommended target based on a result of the lot No. score calculation in the processing 1803 and a result of the expiration date score calculation in the processing 1804. For example, a recommended target score Av is given by the following (Formula 2).

$$Av = m_3 \cdot l + m_4 \cdot d \quad (Formula\ 2)$$

Here, $m_3$ is a weight with respect to the lot No. score 1 represented by a positive constant, and $m_4$ is a weight with respect to the expiration date score d represented by a negative constant. The larger the recommended target score Av, the higher the priority, and the smaller the recommended score Av, the lower the priority. As a result, the recommended target score Av with respect to a total stock of the reagents, the calibrators, and the QC samples in the clinical laboratory 101 is output.

A specific example of processing in the clinical laboratory according to the second embodiment will be described. As in the first embodiment, a case where a remaining amount of an AFP reagent is small in the analysis device 106 (reagent mounting device position 5D) and the reagent replacement is necessary will be described.

After the reagent replacement preparation timing detection unit 206 updates the reagent replacement preparation start notification flag, the notification person-in-charge determination unit 209 performs the processing of the flag confirmation 1303. The processing of the flag confirmation 1303 is performed. From the reagent information management table 214 (see FIG. 3) at this time, it can be confirmed that the reagent replacement preparation start notification flag (column 310) is 1 in the record of the mounted reagent management number (column 302) "20190821-003". The replacement target reagent is the AFP reagent (column 303). Therefore, the management device 102 causes the processing of the recommended replacement reagent determination unit 210 to be executed in an interrupting manner.

The recommended replacement reagent determination unit 210 executes the recommended target determination logic 1801 for the stock of the AFP reagents which are the replacement target reagents. The reagent storage information management table 220 at this time is shown in FIG. 14. From this, it can be seen that the stock of the AFP reagents includes reagents whose storage reagent management numbers are 0002 and 0003 (column 1403).

The lot No. score 1 is calculated for the AFP reagents whose storage reagent management numbers are 0002 and 0003 (lot No. score calculation 1803). Since the lot No. of the reagent requiring the replacement is "20190725-004" (see FIG. 3), the lot No. score 1 of the storage reagent management number 0002 is 1, and the lot No. score 1 of the storage reagent management number 0003 is 0.

Next, the expiration date score d is calculated for the AFP reagents whose storage reagent management numbers are 0002 and 0003 (expiration date score calculation 1804). When the current date is 2019/09/24, the expiration date score d of the storage reagent management number 002 is 9 and the expiration date score d of the storage reagent management number 003 is 8, based on each expiration date (see FIG. 14).

Subsequently, the recommended replacement reagent determination unit 210 performs the recommended target score calculation 1805 to calculate the recommended target score Av. At this time, if the recommended target score Av is calculated using (Formula 2) in which the weight $m_3$ is 3 and the weight $m_4$ is -1, the recommended target scores Av of the AFP reagents whose storage reagent management numbers are 0002 and 0003 are -6 points and -8 points, respectively.

As a result, the storage reagent management number 0002 is specified as the recommended reagent and displayed on the reagent replacement preparation notification screen 1701 (see FIG. 17). Determination of the notification target is performed by executing the processing of the notification person-in-charge determination unit 209 after the recommended reagent is determined. The processing is the same as that of the first embodiment, and thus the description thereof will be omitted. However, in the second embodiment, since the storage places of the reagent, the calibrator, and the QC sample to be replaced are uniquely specified, a value of the person-in-charge position information score p may be a different value.

Although the example in which the recommended replacement reagent is determined in the determination of the person in charge of the reagent replacement preparation is shown here, it is considered that the calibration preparation often precedes the reagent replacement preparation. Therefore, it is desirable to determine the recommended replacement reagent in the determination of the person in charge of the calibration preparation. At this time, when the reagents having the same lot No. are specified as the replacement targets, the notification of the calibration preparation is withheld. After that, upon receiving the completion of the reagent replacement of the reagents having the same lot No. as the replacement target, the calibration preparation start notification flag is updated to 0.

Third Embodiment

In a third embodiment, in addition to the first embodiment, the management device considers a skill level of a laboratory technician when determining an order of task requests to the laboratory technician.

For a laboratory technician who is unfamiliar with a work, such as a newcomer, a time required for reagent replacement, calibration preparation, and QC measurement preparation may be longer than a time required for a skilled laboratory technician. In the third embodiment, a priority of notification is set to be higher for a laboratory technician having a low skill level that requires time for the work. Accordingly, task allocation is executed earlier to a person in charge who may take time for processing, and influence of delay of the processing due to unfamiliarity of the laboratory technician can be reduced.

FIG. 19 shows the person-in-charge skill level management table 223, which is a table for managing the skill level of the laboratory technician working in the clinical laboratory. Column 1902 is a person-in-charge management number, and is a number for uniquely identifying the laboratory technician. Column 1903 indicates a laboratory technician attendance state, in which a case where 1 is registered represents that the laboratory technician is attended to the clinical laboratory, and a case where 0 is registered represents that the laboratory technician is not attended. In column 1903, 0 is registered as an initial value, and when the laboratory technician is attended, start-up of the terminal carried by a PC or the laboratory technician is detected and the value is changed to 1. When the laboratory technician leaves the laboratory, power of the PC or the terminal carried by the laboratory technician is detected to be turned OFF and the value is changed to 0. Column 1904 is a person-in-charge skill level, and a value obtained by dividing the skill level into five stages from 1 to 5 is registered. The skill level 1 is the lowest (most immature) and the skill level 5 is the highest (most proficient). An initial value of the skill level is registered by the administrator in the clinical laboratory. A predicted work average deviation time in column 1904 is an average of differences between standard work times and times required for each laboratory technician to actually finish the work. The standard work time is specifically given by the reagent preparation time (column 404) in the reagent replacement preparation time management table 215 (see FIG. 4), the calibration preparation calibration preparation time (column 504) in the calibration preparation time management table 216 (see FIG. 5), and the QC measurement preparation time (column 604) in the QC measurement preparation time management table 217 (see FIG. 6) .

The management device 102 changes the predicted work average deviation time (column 1905) in the person-in-charge skill level management table 223 such that the person-in-charge skill level (column 1904) is brought close to 3 in an average case, brought close to 1 in a case where much time is required, and brought close to 5 in a case where no time is required, as compared with an average of the laboratory technicians working in the clinical laboratory. For example, assuming that the predicted work deviation time follows a normal distribution, the distribution is divided into 20%, 40%, 60%, 80%, and 100% from a lower side, the skill level of the laboratory technician distributed in 0% to 20% is changed to 1, the skill level of the laboratory technician distributed in 20% to 40% is changed to 2, the skill level of the laboratory technician distributed in 40% to 60% is changed to 3, the skill level of the laboratory technician distributed in 60% to 80% is changed to 4, and the skill level of the laboratory technician distributed in 80% to 100% is changed to 5.

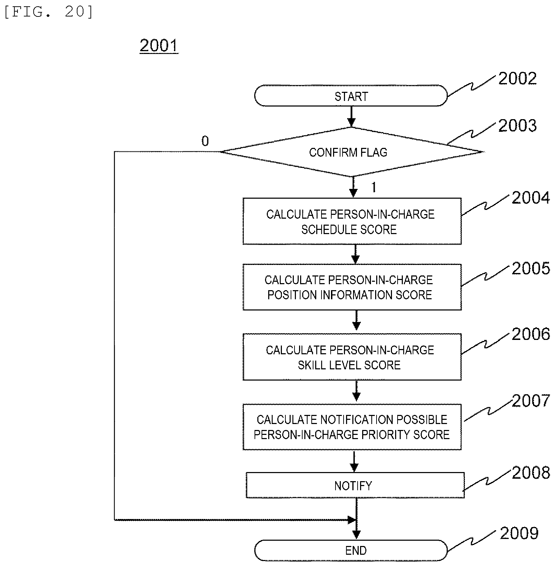

FIG. 20 shows a notification processing logic 2001 executed by the notification person-in-charge determination unit 209 (see FIG. 2). Processing 2003, processing 2004, processing 2005, processing 2007, and processing 2008 are the same as the processing 1303, the processing 1304, processing 1305, the processing 1306, and the processing 1307 in FIG. 13, respectively. Therefore, processing different from the processing in the flowchart of FIG. 13 will be mainly described.

The processing 2006 is processing of person-in-charge skill level score calculation. The management device 102 checks the person-in-charge attendance status (column 1903) in the person-in-charge skill level management table 223 (see FIG. 19), and acquires a list of the attended laboratory technicians having the value of 1. The person-in-charge skill level (column 1904) is checked for the list of the attending laboratory technicians. Here, a value registered in the person-in-charge skill level (column 1904) is output as a person-in-charge skill level score k.

The processing 2007 is processing of performing notification priority score calculation for determining a priority to be notified to the laboratory technician in the clinical laboratory 101. For example, a notification priority score Pr is given by the following (Formula 3).

$$Pr = m_1 \cdot s + m_2 \cdot p + m_5 \cdot k \quad (Formula\ 3)$$

Here, $m_1$ is a weight for the person-in-charge schedule score s represented by a positive constant, $m_2$ is a weight for the person-in-charge position information score p represented by a positive constant, and $m_5$ is a weight for the person-in-charge skill level score k represented by a negative constant. The smaller the notification priority score Pr, the higher the priority, and the larger the notification priority score Pr, the lower the priority. As a result, the notification priority score Pr is output to all the laboratory technicians in the clinical laboratory 101.

A specific example of processing in the clinical laboratory according to the third embodiment will be described. As in the first and second embodiments, a case where a remaining amount of an AFP reagent is small in the analysis device 106 (reagent mounting device position 5D) and the reagent replacement is necessary will be described.

After the reagent replacement preparation timing detection unit 206 updates the reagent replacement preparation start notification flag, the notification person-in-charge determination unit 209 performs the processing of the flag confirmation 1303. From the reagent information management table 214 (see FIG. 3) at this time, it can be confirmed that the reagent replacement preparation start notification flag (column 310) is 1 in the record of the mounted reagent management number (column 302) "20190821-003".

The notification person-in-charge determination unit 209 first performs the processing of person-in-charge schedule score calculation 2004. Since the details are the same as those of the first embodiment, the description thereof will be omitted, and the person-in-charge schedule score s of the laboratory technicians 114 to 117 are 1 point, 5 points, 2 points, and 1 point, respectively.

Subsequently, the processing of the person-in-charge position information score calculation 2005 is performed. Since the details are the same as those of the first embodiment, the person-in-charge position information scores p of the laboratory technicians 114 to 117 are 2.5 points, 0.5 points, 3.5 points, and 3.5 points, respectively. When the recommended target is specified as in the second embodiment, since the position information reference point is uniquely specified, the value of the score p is different from the above.

Next, the notification person-in-charge determination unit 209 performs the processing of the person-in-charge skill level score calculation 2006. The person-in-charge skill level management table 223 at this time is shown in FIG. 19. The attending laboratory technicians are the laboratory technicians P001, P002, P003, and P004. Since the laboratory technicians P001 to P004 correspond to the laboratory technicians 114 to 117, respectively, the person-in-charge skill level scores k of the laboratory technicians 114 to 117 are 3 points, 2 points, 4 points, and 1 point, respectively.

Subsequently, the notification person-in-charge determination unit 209 performs the notification possible person-in-charge priority score calculation 2007 to calculate the notification priority score Pr. At this time, if the notification priority score Pr is calculated using (Formula 3) in which the weight $m_1$ is 1, the weight $m_2$ is 1, and the weight $m_5$ is -1, the notification priority scores Pr of the laboratory technicians 114 to 117 are 0.5 points, 3.5 points, 1.5 points, and 3.5 points, respectively.

As a result, the management device 102 transmits the reagent replacement preparation notification (see FIG. 10) to the terminal 225 carried by the laboratory technician 114 having the lowest score.

Reference Signs List

| | |
|---|---|
| 101: | clinical laboratory |
| 102: | management device |
| 103, 104, 105, 106, 107: | analysis device |
| 108: | pre-processing device |
| 109, 110: | reagent cabinet |
| 111: | calibrator cabinet |

| 112: | QC sample cabinet |
|---|---|
| 113: | work table |
| 114, 115, 116, 117: | laboratory technician |
| 202: | input device |
| 203: | output device |
| 204: | communication device |
| 205: | information processing device |
| 206: | reagent replacement preparation timing detection unit |
| 207: | calibration preparation timing detection unit |
| 208: | QC measurement preparation timing detection unit |
| 209: | notification person-in-charge determination unit |
| 210: | recommended replacement reagent determination unit |
| 211: | recommended calibrator determination unit |
| 212: | recommended QC sample determination unit |
| 213: | storage device |
| 214: | reagent information management table |
| 215: | reagent replacement preparation time management table |
| 216: | calibration preparation time management table |
| 217: | QC measurement preparation time management table |
| 218: | person-in-charge schedule management table |
| 219: | person-in-charge position information management table |
| 220: | reagent storage information management table |
| 221: | calibrator storage information management table |
| 222: | QC sample storage information management table |
| 223: | person-in-charge skill level management table |
| 224: | position information acquisition device |
| 225: | terminal |
| 226: | input device |
| 227: | output device |
| 228: | communication device |
| 229: | notification response device |
| 901: | clinical laboratory map |
| 1001, 1701: | reagent replacement preparation notification screen |
| 1101: | calibration preparation notification screen |
| 1201: | QC measurement preparation notification screen |

**Claims**

1. A management device configured to manage a plurality of analysis devices that analyze a sample in a clinical laboratory provided with the plurality of analysis devices and a cabinet that stores a reagent used for measurement by the plurality of analysis devices or a calibration/quality management sample used for calibration/quality management processing of the reagent, the management device comprising:

a storage device configured to store a reagent information management table that manages a remaining amount of each of in-measurement reagents that are respectively installed on the plurality of analysis devices so as to be used for measurement, a reagent replacement preparation time management table that registers a first preparation time required for preparation for replacing the in-measurement reagent with a storage reagent stored in the cabinet and a calibration/quality management processing preparation time management table that registers a second preparation time required for preparation for calibration/quality management processing of the storage reagent;

a reagent replacement preparation timing detection unit configured to detect a timing to start reagent replacement preparation, based on the remaining amount of the in-measurement reagent managed in the reagent information management table and the first preparation time registered in the reagent replacement preparation time management table;

a calibration/quality management processing preparation timing detection unit configured to detect a timing to start calibration/quality management processing preparation, based on the remaining amount of the in-measurement reagent managed in the reagent information management table and the second preparation time

14

registered in the calibration/quality management processing preparation time management table; and
a notification person-in-charge determination unit configured to notify one of laboratory technicians working in the clinical laboratory of a request for the reagent replacement preparation or the calibration/quality management processing preparation upon receiving detection of a timing by the reagent replacement preparation timing detection unit or the calibration/quality management processing preparation timing detection unit.

2. The management device according to claim 1, wherein

the storage device stores a person-in-charge schedule management table that registers a schedule of each of the laboratory technicians working in the clinical laboratory, and
the notification person-in-charge determination unit is configured to perform prioritization for notifying the request for the reagent replacement preparation or the calibration/quality management processing preparation to the laboratory technicians in the clinical laboratory, based on information on a task currently being performed by each of the laboratory technicians in the clinical laboratory, which is obtained from the person-in-charge schedule management table, and a distance between a position of the cabinet and a current position of the laboratory technician in the clinical laboratory.

3. The management device according to claim 2, wherein

the storage device stores a person-in-charge skill level management table that registers a skill level of each of the laboratory technicians working in the clinical laboratory, and
the notification person-in-charge determination unit is configured to perform prioritization for notifying the request for the reagent replacement preparation or the calibration/quality management processing preparation to the laboratory technicians in the clinical laboratory, based on information on the task currently being performed by each of the laboratory technicians in the clinical laboratory, which is obtained from the person-in-charge schedule management table, the distance between the position of the cabinet and the current position of the laboratory technician in the clinical laboratory, and the skill level of the laboratory technician in the laboratory obtained from the person-in-charge skill level management table.

4. The management device according to claim 1, further comprising:

a recommended replacement reagent determination unit configured to determine a reagent recommended as the storage reagent among the reagents stored in the cabinet, wherein
the storage device stores a reagent storage information management table that registers reagent information including a lot number and an expiration date of the reagent stored in the cabinet, and
the recommended replacement reagent determination unit determines the reagent recommended as the storage reagent, based on the lot number of each of the in-measurement reagents managed in the reagent information management table, and the lot number and the expiration date of the reagent obtained from the reagent storage information management table.

5. The management device according to claim 4, wherein

the calibration/quality management processing includes calibration of the storage reagent, and
when the recommended replacement reagent determination unit determines a reagent having the same lot number as one of the in-measurement reagents as the storage reagent, the request for calibration preparation of the storage reagent is unnecessary.

6. The management device according to claim 4, wherein

the storage device stores a person-in-charge schedule management table that registers a schedule of each of the laboratory technicians working in the clinical laboratory, and
the notification person-in-charge determination unit is configured to perform prioritization for notifying the request for the reagent replacement preparation to each of the laboratory technicians in the clinical laboratory, based on information on a task currently being performed by the laboratory technician in the clinical laboratory, which is obtained from the person-in-charge schedule management table, and a distance between a position of the cabinet that stores the storage reagent recommended by the recommended replacement reagent determination unit and a current position of the laboratory technician in the clinical laboratory.

7. The management device according to claim 1, further comprising:

a recommended calibration/quality management sample determination unit configured to determines a calibration/quality management sample recommended as a calibration/quality management sample to be used for calibration/quality management processing of the storage reagent among calibration/quality management samples stored in the cabinet, wherein
the storage device stores a calibration/quality management sample storage information management table that registers calibration/quality management sample information including an expiration date of each of the calibration/quality management samples stored in the cabinet, and
the recommended calibration/quality management sample determination unit determines the recommended calibration/quality management sample based on the expiration date of each of the calibration/quality management samples obtained from the calibration/quality management sample storage information management table.

8. The management device according to claim 7, wherein

the storage device stores a person-in-charge schedule management table that registers a schedule of each of the laboratory technicians working in the clinical laboratory, and
the notification person-in-charge determination unit is configured to perform prioritization for notifying the request for the calibration/quality management processing preparation to the laboratory technicians in the clinical laboratory, based on information on a task currently being performed by each of the laboratory technicians in the clinical laboratory, which is obtained from the person-in-charge schedule management table, and a distance between a position of the cabinet that stores the calibration/quality management sample recommended by the recommended calibration/quality management sample determination unit and a current position of the laboratory technician in the clinical laboratory.

9. The management device according to claim 1, wherein
the calibration/quality management processing is calibration of the storage reagent using a calibrator as the calibration/quality management sample, or QC measurement of the storage reagent using a QC sample as the calibration/quality management sample.

10. A request method for reagent replacement preparation or calibration/quality management processing preparation in a clinical laboratory provided with a plurality of analysis devices that analyze a sample and a cabinet that stores a reagent used for measurement by the plurality of analysis devices or a calibration/quality management sample used for calibration/quality management processing of the reagent, the request method comprising:

storing, in advance, a reagent information management table that manages a remaining amount of each of in-measurement reagents that are respectively installed on the plurality of analysis devices so as to be used for measurement, a reagent replacement preparation time management table that registers a first preparation time required for preparation for replacing the in-measurement reagent with a storage reagent stored in the cabinet and a calibration/quality management processing preparation time management table that registers a second preparation time required for preparation for calibration/quality management processing of the storage reagent;
detecting a first timing to start reagent replacement preparation based on the remaining amount of the in-measurement reagent managed in the reagent information management table and the first preparation time registered in the reagent replacement preparation time management table;
detecting a second timing to start calibration/quality management processing preparation based on the remaining amount of the in-measurement reagent managed in the reagent information management table and the second preparation time registered in the calibration/quality management processing preparation time management table; and
notifying one of laboratory technicians working in the clinical laboratory of a request for the reagent replacement preparation upon receiving detection of the first timing, and a request for the calibration/quality management processing preparation by receiving detection of the second timing.

11. The request method according to claim 10, comprising:

storing, in advance, a person-in-charge schedule management table that registers a schedule of each of the laboratory technicians working in the clinical laboratory; and
performing prioritization for notifying the request for the reagent replacement preparation or the calibration/quality management processing preparation to the laboratory technicians in the clinical laboratory, based on information on a task currently being performed by each of the laboratory technicians in the clinical laboratory, which is obtained from the person-in-charge schedule management table, and a distance between a position of the cabinet and a current position of the laboratory technician in the clinical laboratory, and notifying the request to one of the laboratory technicians that has a highest priority.

12. The request method according to claim 11, comprising:

   storing, in advance, the person-in-charge skill level management table that registers a skill level of each of the laboratory technicians working in the clinical laboratory, and
   performing the prioritization for notifying the request for the reagent replacement preparation or the calibration/quality management processing preparation to the laboratory technicians in the clinical laboratory based on information on the task currently being performed by each of the laboratory technicians in the clinical laboratory, which is obtained from the person-in-charge schedule management table, the distance between the position of the cabinet and the current position of the laboratory technician in the clinical laboratory, and the skill level of the laboratory technician in the laboratory obtained from the person-in-charge skill level management table, and notifying the request to one of the laboratory technicians that has a highest priority.

13. The request method according to claim 10, comprising:

   storing, in advance, a reagent storage information management table that registers reagent information including a lot number and an expiration date of the reagent stored in the cabinet, and
   determining the reagent recommended as the storage reagent among the reagents stored in the cabinet, based on the lot number of each of the in-measurement reagents managed in the reagent information management table, and the lot number and expiration date of the reagent obtained from the reagent storage information management table, and notifying the reagent with the request for the reagent replacement preparation.

14. The request method according to claim 13, wherein

   the calibration/quality management processing includes calibration of the storage reagent, and
   when the recommended replacement reagent determination unit determines a reagent having the same lot number as one of the in-measurement reagents as the storage reagent, the request for calibration preparation of the storage reagent is unnecessary.

15. The request method according to claim 10, wherein
   the calibration/quality management processing is calibration of the storage reagent using a calibrator as the calibration/quality management sample, or QC measurement of the storage reagent using a QC sample as the calibration/quality management sample.

[FIG. 1]

[FIG. 2]

102

| MANAGEMENT DEVICE | 230 | POSITION INFORMATION ACQUISITION DEVICE | 224 |

202

203 — INPUT DEVICE
231 — ANALYSIS DEVICE/PRE-PROCESSING DEVICE — 103~108

204 — OUTPUT DEVICE
225

205 — COMMUNICATION DEVICE — 232 — TERMINAL

INFORMATION PROCESSING DEVICE

| 206 — REAGENT REPLACEMENT PREPARATION TIMING DETECTION UNIT | CALIBRATION PREPARATION TIMING DETECTION UNIT — 207 |
| 208 — QC MEASUREMENT PREPARATION TIMING DETECTION UNIT | NOTIFICATION PERSON-IN-CHARGE DETERMINATION UNIT — 209 |
| 210 — RECOMMENDED REPLACEMENT REAGENT DETERMINATION UNIT | RECOMMENDED CALIBRATOR DETERMINATION UNIT — 211 |
| 212 — RECOMMENDED QC SAMPLE DETERMINATION UNIT | |

TERMINAL
226 — INPUT DEVICE
227 — OUTPUT DEVICE
228 — COMMUNICATION DEVICE
229 — NOTIFICATION RESPONSE DEVICE

STORAGE DEVICE

| 214 — REAGENT INFORMATION MANAGEMENT TABLE | REAGENT REPLACEMENT PREPARATION TIME MANAGEMENT TABLE — 215 |
| 216 — CALIBRATION PREPARATION TIME MANAGEMENT TABLE | QC MEASUREMENT PREPARATION TIME MANAGEMENT TABLE — 217 |
| 218 — PERSON-IN-CHARGE SCHEDULE MANAGEMENT TABLE | PERSON-IN-CHARGE POSITION INFORMATION MANAGEMENT TABLE — 219 |
| 220 — REAGENT STORAGE INFORMATION MANAGEMENT TABLE | CALIBRATOR STORAGE INFORMATION MANAGEMENT TABLE — 221 |
| 222 — QC SAMPLE STORAGE INFORMATION MANAGEMENT TABLE | PERSON-IN-CHARGE SKILL LEVEL MANAGEMENT TABLE — 223 |

213

19

EP 4 116 900 A1

[FIG. 3]

| MOUNTED REAGENT MANAGEMENT NUMBER (302) | REAGENT NAME (303) | REAGENT LOT NO. (304) | REAGENT MOUNTING DEVICE POSITION (305) | EXPIRATION DATE (306) | REMAINING AMOUNT (307) | NUMBER OF TIME OF REMAINING MEASUREMENT (308) | REAGENT OUT PREDICTION TIME POINT (309) | REAGENT REPLACEMENT PREPARATION START NOTIFICATION FLAG (310) | CALIBRATION PREPARATION START NOTIFICATION FLAG (311) | QC MEASUREMENT PREPARATION START NOTIFICATION FLAG (312) |
|---|---|---|---|---|---|---|---|---|---|---|
| 20190821-001 | ALB | 20190725-001 | 3B | 2019/09/30 | 10ml | 10 TIMES | 2019/09/24 15:00:00 | 0 | 1 | 0 |
| 20190821-002 | ALB | 20190725-001 | 3B | 2019/09/30 | 40ml | 40 TIMES | NULL | 0 | 0 | 0 |
| 20190821-003 | AFP | 20190725-004 | 5D | 2019/10/02 | 5ml | 5 TIMES | 2019/09/24 14:20:00 | 1 | 1 | 1 |
| 20190903-001 | AFP | 20190726-001 | 3D | 2019/10/03 | 30ml | 30 TIMES | NULL | 0 | 0 | 0 |
| 20190903-002 | TP | 20190726-002 | 4B | 2019/10/03 | 50ml | 50 TIMES | NULL | 0 | 0 | 0 |
| 20190903-003 | KOH | 20190815-001 | 5D | 2019/09/29 | 20ml | 20 TIMES | NULL | 0 | 0 | 0 |
| : | : | : | : | : | : | : | : | : | : | : |

EP 4 116 900 A1

[FIG. 4]

215

| REAGENT MANAGEMENT NUMBER | REAGENT NAME | REAGENT REPLACEMENT PREPARATION TIME | WORK DIFFICULTY |
|---|---|---|---|
| 0001 | ALB | 10min | 1 |
| 0002 | AFP | 10min | 1 |
| 0003 | TP | 10min | 1 |
| 0004 | KOH | 10min | 1 |
| : | : | : | : |

402  403  404  405

[FIG. 5]

216

| CALIBRATOR MANAGEMENT NUMBER | CALIBRATOR NAME | CALIBRATION PREPARATION TIME | WORK DIFFICULTY |
|---|---|---|---|
| 0001 | ALB-C | 40min | 2 |
| 0002 | AFP-C | 35min | 2 |
| 0003 | TP-C | 30min | 2 |
| 0004 | KOH-C | 40min | 2 |
| : | : | : | : |

502  503  504  505

[FIG. 6]

<u>217</u>

| QC SAMPLE MANAGEMENT NUMBER | QC SAMPLE NAME | QC MEASUREMENT PREPARATION TIME | WORK DIFFICULTY |
|---|---|---|---|
| 0001 | ALB-Q | 20min | 1 |
| 0002 | AFP-Q | 35min | 2 |
| 0003 | TP-Q | 10min | 1 |
| 0004 | KOH-Q | 40min | 2 |
| : | : | : | : |

602  603  604  605

[FIG. 7]

| TASK ID | PERSON-IN-CHARGE MANAGEMENT NUMBER | WORK CONTENT | WORK DIFFICULTY | TASK START TIME | COMPLETION FLAG |
|---|---|---|---|---|---|
| 001 | P001 | SAMPLE RECEPTION | 5 | 2019/09/23-08:20:00 | 1 |
| 002 | P002 | SAMPLE RECEPTION | 5 | 2019/09/23-09:30:00 | 0 |
| 003 | P003 | ANALYSIS PREPARATION | 2 | 2019/09/23-13:15:00 | 0 |
| 004 | P004 | DEVICE CHECK | 3 | NULL | 0 |
| : | : | : | : | : | : |

702  703  704  705  706  707

[FIG. 8]

219

|  | 802 | 803 |
|---|---|---|
|  | PERSON-IN-CHARGE MANAGEMENT NUMBER | CLINICAL LABORATORY MAP CURRENT POSITION |
|  | P001 | 4C |
|  | P002 | 8C |
|  | P003 | 10C |
|  | P004 | 9E |
|  | : | : |

[FIG. 9]

901

[FIG. 10]

REAGENT REPLACEMENT PREPARATION NOTIFICATION

1001

CONTENT: AFP REAGENT REMAINING MEASUREMENT 5 TIMES

PROCESSING METHOD:
PLEASE REPLACE AFP REAGENT

REPLACEMENT TARGET DEVICE:
ANALYSIS DEVICE 5D (POSITION 5D)

REAGENT STORAGE PLACE:
REAGENT CABINET
(POSITION 7B)

PROCESS THIS NOTIFICATION?

YES                    NO

1002                  1003

[FIG. 11]

CALIBRATION PREPARATION NOTIFICATION _____ 1101

CONTENT: AFP REAGENT REMAINING MEASUREMENT 5 TIMES

PROCESSING METHOD:
PLEASE PERFORM AFP REAGENT CALIBRATION PREPARATION

REPLACEMENT TARGET DEVICE:
    ANALYSIS DEVICE 5D (POSITION 5D)

REAGENT STORAGE PLACE:
    CALIBRATOR CABINET
    (POSITION 10B)

PROCESS THIS NOTIFICATION?

| YES | NO |
|-----|----|

1102          1103

[FIG. 12]

QC MEASUREMENT PREPARATION NOTIFICATION

1201

CONTENT: AFP REAGENT REMAINING MEASUREMENT 5 TIMES

PROCESSING METHOD:
PLEASE PERFORM AFP REAGENT QC MEASUREMENT PREPARATION

REPLACEMENT TARGET DEVICE:
  ANALYSIS DEVICE 5D (POSITION 5D)

REAGENT STORAGE PLACE:
  CALIBRATOR CABINET
  (POSITION 11B)

PROCESS THIS NOTIFICATION?

| YES | NO |

1202       1203

[FIG. 13]

1301

[FIG. 14]

220

| STORAGE REAGENT MANAGEMENT NUMBER | REAGENT NAME | REAGENT LOT NO. | EXPIRATION DATE | STORAGE PLACE |
|---|---|---|---|---|
| 0001 | ALB | 20190725-002 | 2019/09/30 | 7B |
| 0002 | AFP | 20190725-004 | 2019/10/02 | 7B |
| 0003 | AFP | 20190724-002 | 2019/10/01 | 7B |
| 0004 | TP | 20190727-003 | 2019/10/04 | 8B |
| 0005 | KOH | 20190815-001 | 2019/09/29 | 8B |
| ⋮ | ⋮ | ⋮ | ⋮ | ⋮ |

1402    1403    1404    1405    1406

[FIG. 15]

221

| STORAGE CALIBRATOR MANAGEMENT NUMBER | CALIBRATOR NAME | EXPIRATION DATE | STORAGE PLACE |
|---|---|---|---|
| 0001 | ALB-C | 2019/09/30 | 10B |
| 0002 | AFP-C | 2019/10/02 | 10B |
| 0003 | AFP-C | 2019/10/01 | 10B |
| 0004 | TP-C | 2019/10/04 | 10B |
| 0005 | KOH-C | 2019/09/29 | 10B |
| : | : | : | : |

1502   1503   1504   1505

[FIG. 16]

222

| STORAGE QC SAMPLE MANAGEMENT NUMBER | QC SAMPLE NAME | EXPIRATION DATE | STORAGE PLACE |
|---|---|---|---|
| 0001 | ALB-Q | 2019/09/30 | 11B |
| 0002 | AFP-Q | 2019/10/02 | 11B |
| 0003 | AFP-Q | 2019/10/01 | 11B |
| 0004 | TP-Q | 2019/10/04 | 11B |
| 0005 | KOH-Q | 2019/09/29 | 11B |
| : | : | : | : |

1602   1603   1604   1605

[FIG. 17]

REAGENT REPLACEMENT PREPARATION NOTIFICATION

1701

CONTENT: AFP REAGENT REMAINING MEASUREMENT 5 TIMES

PROCESSING METHOD:
PLEASE REPLACE AFP REAGENT

REPLACEMENT TARGET DEVICE:
ANALYSIS DEVICE 5D (POSITION 5D)

REAGENT STORAGE PLACE:
REAGENT CABINET
(POSITION 7B)

1702

RECOMMENDED REPLACEMENT REAGENT: AFP
LOT NO.: 20190725-004
REAGENT STORAGE PLACE:7B

PROCESS THIS NOTIFICATION?

YES          NO

1002          1003

[FIG. 18]

1801

```
         ┌─────────────────────────┐
         │          START          │ ──── 1802
         └─────────────────────────┘
                      │
                      ▼
         ┌─────────────────────────┐
         │   CALCULATE LOT NO. SCORE │ ──── 1803
         └─────────────────────────┘
                      │
                      ▼
         ┌─────────────────────────┐
         │ CALCULATE EXPIRATION DATE SCORE │ ──── 1804
         └─────────────────────────┘
                      │
                      ▼
         ┌─────────────────────────┐
         │   CALCULATE RECOMMENDED   │ ──── 1805
         │  REPLACEMENT TARGET SCORE │
         └─────────────────────────┘
                      │
                      ▼
         ┌─────────────────────────┐
         │           END           │ ──── 1806
         └─────────────────────────┘
```

[FIG. 19]

223

| PERSON-IN-CHARGE MANAGEMENT NUMBER | PERSON-IN-CHARGE ATTENDANCE STATUS | PERSON-IN-CHARGE SKILL LEVEL | PREDICTED WORK AVERAGE DEVIATION TIME |
|---|---|---|---|
| | 1902 | 1903 | 1904 | 1905 |
| P001 | 1 | 3 | 2min. |
| P002 | 1 | 2 | 7min. |
| P003 | 1 | 4 | 1min. |
| P004 | 1 | 1 | 10min. |
| P005 | 0 | 5 | 0min. |
| : | : | : | |

[FIG. 20]

2001

```
        ┌──────────────────────┐
        │        START         │───╮ 2002
        └──────────┬───────────┘
                   │
          0   ╱────┴────╲
     ┌────────   CONFIRM FLAG   ╲───╮ 2003
     │        ╲────┬────╱
     │             │ 1
     │    ┌────────┴──────────┐
     │    │ CALCULATE PERSON-IN-CHARGE │──╮ 2004
     │    │   SCHEDULE SCORE   │
     │    └────────┬──────────┘
     │             │
     │    ┌────────┴──────────┐
     │    │ CALCULATE PERSON-IN-CHARGE │──╮ 2005
     │    │ POSITION INFORMATION SCORE │
     │    └────────┬──────────┘
     │             │
     │    ┌────────┴──────────┐
     │    │ CALCULATE PERSON-IN-CHARGE │──╮ 2006
     │    │   SKILL LEVEL SCORE │
     │    └────────┬──────────┘
     │             │
     │    ┌────────┴──────────┐
     │    │ CALCULATE NOTIFICATION POSSIBLE │──╮ 2007
     │    │ PERSON-IN-CHARGE PRIORITY SCORE │
     │    └────────┬──────────┘
     │             │
     │    ┌────────┴──────────┐
     │    │       NOTIFY       │──╮ 2008
     │    └────────┬──────────┘
     │             │
     └─────────────┤
        ┌──────────┴───────────┐
        │         END          │───╮ 2009
        └──────────────────────┘
```

**INTERNATIONAL SEARCH REPORT**

| International application No. |
| --- |
| PCT/JP2021/001671 |

A. CLASSIFICATION OF SUBJECT MATTER
Int.Cl. G06Q10/06(2012.01)i, G01N35/00(2006.01)i
FI: G06Q10/06, G01N35/00A, G01N35/00C

According to International Patent Classification (IPC) or to both national classification and IPC

B. FIELDS SEARCHED

Minimum documentation searched (classification system followed by classification symbols)
Int.Cl. G06Q10/06, G01N35/00

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2009-162584 A (OLYMPUS CORPORATION) 23 July 2009 (2009-07-23), entire text, all drawings | 1-15 |
| A | JP 2009-168730 A (HITACHI HIGH-TECHNOLOGIES CORP.) 30 July 2009 (2009-07-30), entire text, all drawings | 1-15 |
| A | WO 2013/065528 A1 (HITACHI HIGH-TECHNOLOGIES CORP.) 10 May 2013 (2013-05-10), entire text, all drawings | 1-15 |
| A | JP 2007-198986 A (SHIMADZU CORPORATION) 09 August 2007 (2007-08-09), entire text, all drawings | 1-15 |
| A | WO 2019/064398 A1 (MITSUBISHI HEAVY INDUSTRIES MACHINERY SYSTEMS LTD.) 04 April 2019 (2019-04-04), entire text, all drawings | 1-15 |

☐ Further documents are listed in the continuation of Box C.   ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 08 April 2021 | 20 April 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/001671

| | | |
|---|---|---|
| JP 2009-162584 A | 23 July 2009 | (Family: none) |
| JP 2009-168730 A | 30 July 2009 | (Family: none) |
| WO 2013/065528 A1 | 10 May 2013 | US 2014/0250339 A1<br>entire text, all drawings<br>EP 2775307 A1<br>CN 103907027 A |
| JP 2007-198986 A | 09 August 2007 | (Family: none) |
| WO 2019/064398 A1 | 04 April 2019 | EP 3499450 A1<br>entire text, all drawings<br>KR 10-2019-0056357 A<br>CN 109844795 A |

Form PCT/ISA/210 (patent family annex) (January 2015)

**REFERENCES CITED IN THE DESCRIPTION**

**Patent documents cited in the description**

- JP 2019045446 A
- JP 2004271265 A